# EUROPEAN PATENT SPECIFICATION

(11) **EP 3 006 052 B1**
(45) Date of publication and mention of the grant of the patent: **11.07.2018**
(21) Application number: 14807148.3
(22) Date of filing: 22.04.2014
(51) Int. Cl.: A61L 2/07, B08B 3/10, B01L 3/00, G01N 33/00

(54) **METHOD FOR CLEANING DISSOLUTION VESSELS AND SUBSEQUENT DOSING OF A DISSOLUTION MEDIUM, AND MOBILE MODULAR CLEANING AND DOSING EQUIPMENT FOR THE IMPLEMENTATION THEREOF**
VERFAHREN ZUR REINIGUNG VON AUFLÖSUNGSBEHÄLTERN UND ANSCHLIESSENDEM DOSIERUNG EINES AUFLÖSUNGSMEDIUMS UND MOBILE MODULARE REINIGUNGS- UND DOSIERVORRICHTUNG ZUR DURCHFÜHRUNG
PROCÉDÉ DE NETTOYAGE DE RÉCIPIENTS DE DISSOLUTION ET DE DOSAGE POSTÉRIEUR DE MILIEU DE DISSOLUTION EN VERRE, ET DISPOSITIF MODULAIRE MOBILE DE NETTOYAGE ET DE DOSAGE POUR SA MISE EN OEUVRE

(30) Priority: 03.06.2013 ES 201330809
(43) Date of publication of application: 13.04.2016
(73) Proprietor: Borrego Castro, Manuel, 08203 Barcelona (ES)
(72) Inventor: Borrego Castro, Manuel, 08203 Barcelona (ES)
(74) Representative: Isern Patentes y Marcas S.L.
(86) International application number: PCT/ES2014/070345
(87) International publication number: WO 2014/195543

(56) References cited:
- EP-A2- 1 256 378
- WO-A1-01/02095
- WO-A1-2006/043935
- WO-A1-2013/137325
- ES-T3- 2 393 569
- US-A- 4 014 357
- US-A- 4 318 885
- US-A- 4 635 665
- US-A- 5 741 237
- US-A1- 2003 198 125
- US-A1- 2010 037 919
- US-A1- 2010 037 919
- US-B1- 6 652 495
- DATABASE WPI Week 197714, Derwent Publications Ltd., London, GB; Class P43, AN 1977-C9152Y, XP055299564 'STEAMER FOR CLEANING BEAKER VESSELS - HAS STEAM UNDER PRESSURE MIXED WITH SOLVENT METERED THROUGH VALVE' & US 4 014 357 A (SNEED A.L.) 29 March 1977

## Description

### Object of the invention

The object of the present invention is to provide a procedure or method for cleaning dissolution vessels using steam and the subsequent filling of said dissolution vessels with a dissolution medium or dissolvent ready for use, the dissolvent, before being dosed, will be preheated and the appropriate amount will be measured. The mobile modular equipment for the implementation of the aforementioned method is also the object of the present invention.

### Background to the invention

Dissolution testing is becoming more and more common in the pharmaceutical industry. These tests determine the amount of active ingredient that is released into a certain dissolution medium within an established timeframe and set temperature. The active ingredient is normally contained in a type of pharmaceutical formula such as tablets, film-coated tablets, capsules or similar products used in therapeutic care.

The dissolution testing is carried out in a receptacle generally referred to as a dissolution glass that contains a pre-set amount of dissolution medium at a previously chosen temperature, normally 37 degrees, in which a dosing unit of the therapeutic agent that contains the active ingredient(s) is added and stirred. The samples of the dissolution are removed at pre-set time intervals and said samples are then analysed in suitable equipment in order to obtain the amount of the active ingredient that has been released into the dissolution medium. When the testing is finished, the equipment must be cleaned to eliminate all trace or residue of the dissolution in order to move onto a new test of the same therapeutic agent or a different one.

The foregoing method may be performed manually or using equipment with a greater or lesser level of automation.

According to the state of the art, the washing or cleaning of dissolution vessels is performed using liquids that are subsequently sucked up and brushes are occasionally used to obtain the desired results.

The proposed invention offers a method for cleaning dissolution vessels using pressurised steam and their subsequent filling with a new dissolution medium and mobile equipment to carry out the method for one or more dissolution vessels without having to remove them from their housing in the dissolution equipment and without using equipment that consumes a large amount of water and time in cleaning and subsequently conditioning the dissolution vessels.

It is known from US 6652495 a system for disposing of body fluids collected during surgery comprises a canister and an apparatus for emptying and cleaning the canister.

Also, US 5741237 discloses a system for collecting and disposing of body fluids collected during surgery comprises a canister and a servicing unit for removing the body fluids from the canister and cleaning the canister for reuse.

The above cited documents are mentioned in this application by reference.

### Description of the invention

The proposed method for cleaning dissolution vessels and subsequent dosing of the dissolution medium comprises the following stages:
A. Standard conditioning, which in turn comprises the successive or simultaneous sub-stages:
   a. Generating steam
   b. Preheating the dissolution medium to be dosed.
   c. Emptying the vacuum tanks and optional insertion of neutralising or anti-foam solution into said vacuum tanks.
B. Cleaning the dissolution glass, which in turn comprises the following successive or simultaneous sub-stages:
   a. Fitting and adjusting the lid of the dissolution glass.
   b. Pressing the button to start the process
   c. Sucking up the dissolution medium using the suction tube.
   d. Decanting into the vacuum tank.
   e. Optionally adding steam of one or more chemical additives
   f. Introducing steam at predetermined temperatures and pressure levels for a given time period depending on the dissolution medium used and the therapeutic agent tested
   g. Sucking up steam using the suction tube and decanting into the vacuum tank.
   h. Decanting the dissolution medium and steam contained in the vacuum tank to the outside.
C. Dosing, which in turn comprises the successive or simultaneous sub-stages:
   a. Decanting the preheated dissolution medium from the storage deposit into the dosing deposit.
   b. Determining the predetermined amount of dissolution medium to be dosed using a gravimetric measuring device.
   c. Decanting the dissolution medium into the dissolution glass.

Whereby, stage B, cleaning the dissolution glass, and stage C, dosing, are consecutive and independent of each other and that in the procedure described above, stage B is compulsory, since during the dissolution testing, the final stage is to leave the already used dissolution vessels ready for carrying out new tests, which may be required after a certain time has elapsed, and stage C which is an optional part of the procedure.

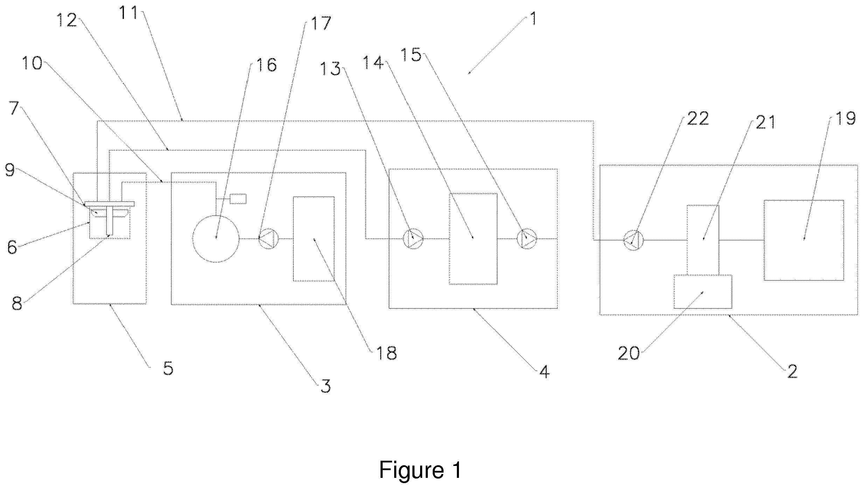

The modular cleaning and dosing equipment for the implementation of the aforementioned method comprises the following modules, provided in figure 1:
A. Lid module, which in turn comprises a lid consisting of a piece manufactured from plastic with coupling and seal-tight adjustment means, preferably of the sealing gasket type, at the mouth of the dissolution glass; a vacuum breaker system conveniently arranged in the lid, preferably of the hole type, which enables the insertion of a plastic filter, preferably a 35 micron mesh made of PVDF (polyvinylidene difluoride); a suction tube of variable length, manufactured from a plastic material, in order to be used in different sized dissolution vessels, coupled in a seal-tight manner to the lid and connected to the suction route of the dissolution medium; a non-return valve, preferably of the ball and spring type, suitably arranged in the suction tube in order to prevent the dissolution medium that has been sucked up accidently returning to the dissolution glass; a particle filter suitably arranged in said suction tube, preferably a mesh manufactured from stainless steel; a supply route for steam connected to a steam distributor that consists of a piece made from from plastic, preferably PTFE (polytetrafluorethylene), with upper seals ideally manufactured by Viton, a registered brand of fluoroelastomer, an inner channel for the circulation of steam and a series of holes arranged perpendicularly to the channel with outer opening so that the steam can be radially supplied against the inner walls of the dissolution glass; a supply route for the dissolution medium and an activation switch to give the command to the control module to commence or detain the process.
B. Suction module for the dissolution medium and steam that comprises a sucking system, preferably a suction pump with a sealing gasket, connected to a vacuum tank for the storage of already used dissolution medium and steam, equipped with level sensors and vacuum pumps for the same with check valves and their corresponding interconnection and connection piping and fittings to enable connection with other modules.
C. Steam generation module that comprises a boiler manufactured from stainless steel duly equipped with one or more electrical resistances, water level sensors, safety thermostat, pressure/temperature control system, preferably but not limited to, via pressure switch, solenoid valve, safety valve, vacuum breaker system, preferably of the vent valve model, filling pump for the boiler with its corresponding anti-return valve, water supply tank for the boiler with level sensors, as well as their corresponding interconnection and connection piping and fittings to connect the rest of the modules, and, as an optional design element, a device for adding chemical additives to the supply route of the generated steam.
D. Dosing module for the dissolution medium that contains a storage deposit for the dissolution medium with level sensors and heating system with temperature control, preferably one or more electrical resistances with thermostats; gravimetric measuring device for the dissolution medium to be supplied, preferably using gravimetric load cells; dosing deposit that contains the amount of dissolution medium to be supplied into the dissolution glass incorporating a vacuum breaker system, preferably of the vent valve type; a drive system for the dissolution medium for dosing, and their corresponding tubing, fittings and solenoid for interconnecting and connecting the rest of the modules,
E. Control module with an information screen and data input that controls, at least, the activation of the switch for the lid module, the steam pressure, the insertion time for the steam in the dissolution glass, the time and cycles to suck up the dissolution medium, the temperature and amount of the dissolution medium to be dosed, and, optionally, the amount of chemical additive to be added to the steam to be supplied
F. Transport module that comprises a frame to which the rest of the modules are coupled, and on which transportation means are suitably arranged, preferably groups of one or more wheels.

As a design option, the different modules that comprise the invention may be separated from each other or grouped in different module combinations, each module or group of modules being provided with its corresponding frame and transport means, preferably in groups of one or more wheels.

### Description of the figures

Figure 1: diagram of the modular equipment
Figure 2 a: top view of the steam distributor

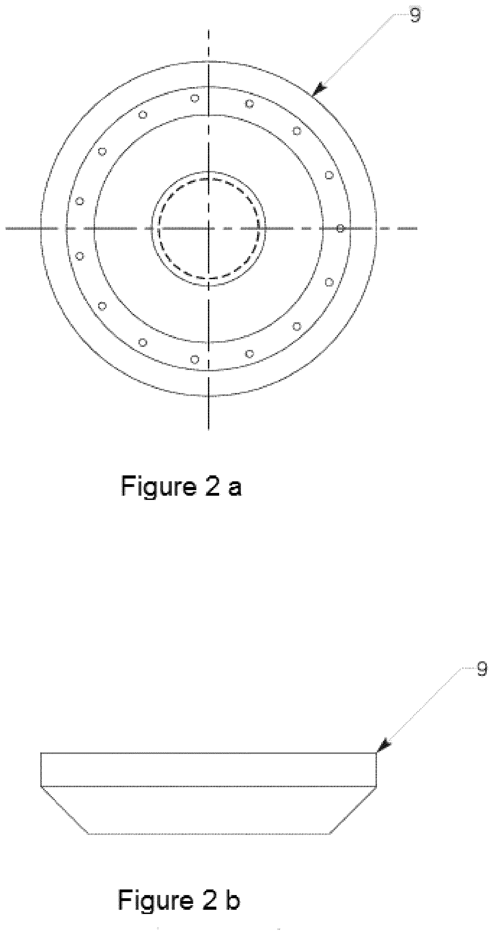

Figure 2 b: side view of the steam distributor

### List of references

1. Modular cleaning and dosing equipment for dissolution vessels.
2. Dosing module
3. Steam generation module
4. Suction module
5. Lid module
6. Dissolution glass
7. Lid
8. Suction tube
9. Steam distributor
10. Steam supply route
11. Dissolution medium supply route
12. Suction route
13. Suction pump
14. Vacuum tank
15. Vacuum pump
16. Boiler
17. Filling pump
18. Supply deposit to the boiler
19. Dissolution material deposit
20. Gravimetric measuring device
21. Dosing deposit
22. Dosing pump

### Preferred embodiment of the invention

Figure 1 shows a preferred embodiment of the mobile modular equipment for cleaning and dosing dissolution vessels in which the suction module and steam generation module are joined to the same assembly and separated from the dosing and lid modules. In this embodiment, the module interconnection tubing, along with quick coupling fittings and wheeled transport means for the module assembly are conveniently arranged.

Also, as can see in figure 1, this mobile modular equipment comprises a cover module (5), which in turn comprises a cover (7) with coupling and seal-tight adjustment means at the top of the dissolution vessel (6); a vacuum breaker system, being said vacuum breaker system an opening arranged in the cover (7), which enables the insertion of a filter. An aspiration tube (8) of variable length is coupled in a seal-tight manner to the cover (7) and connected to the aspiration line (12) of the dissolution media. Furthermore, cover module (5) includes a check valve suitably arranged in the aspiration tube (8), a particle filter suitably arranged in the aspiration tube (8), a supply line for steam connected to a steam distributor (9) that consists of a piece with upper seals, an inner channel for the circulation of steam and a series of holes arranged perpendicularly to the channel with outer openings, and a supply line for the dissolution media and an activation switch.

This equipment requires general conditioning prior to the start of the cleaning and dosing method, consisting of the preparing steam via the supply of water to the boiler from the filling deposit using the filling pump (17); preheating, preferably using electrical resistance, the water until it reaches its evaporates and overheating up to a pre-set minimum pressure level indicated by the sensor; and optionally, introducing a neutraliser/anti-foam solution to condition the dissolution medium, inside of the vacuum tank, to sucked up. Likewise, the dissolution medium to be dosed must preferably be preheated using electrical resistance to the pre-set temperature.

The washing and dosing method in this embodiment are carried out independently of each other.

The method begins with the placement of the lid in the mouth of the dissolution glass and then actuating the manual switch to indicate to the control module to start the process.

The washing method necessarily implies sucking up the dissolution medium present in the dissolution glass through the suction tube and decanting it into the vacuum pump (22), preferably being inserted through the lower portion of the same, which is mixed with the medium present in the tank and, optionally, with the neutralising and/or anti-foam solution previously added during the conditioning stage.

After the washing thereof, steam from the boiler is added to the dissolution glass through the steam route joined to the steam distributor, figure 2 a and 2 b, which enables it to be radially and symmetrically projected onto the walls of the glass so that it may be cleaned better. The steam is sucked up using the suction tube and taken to the vacuum tank via the suction route, such that the cleaning process has been carried out.

When the level of the vacuum tank exceeds a limit predetermined by the maximum level sensor, the vacuum tank, through the vacuum pump, expels the already used dissolution medium, sending it the suitable storage and waste treatment devices. The end of the vacuum process is determined using a minimum level sensor of the vacuum tank.

When the water level in the boiler exceeds the pre-set limit, the filling pump of the boiler will replace the water and start the conditioning process once again.

The method for dosing the dissolution medium begins with decanting the pre-set amount of the medium, previously heated in the conditioning process, to the dosing deposit, preferably determined by gravimetric load cell measurement devices, said decanting to the dissolution glass is performed using the dosing pump (22).

When the level of the dissolution medium present in the medium tank exceeds a predetermined lower limit, it will be necessary to replace the dissolution medium and begin the conditioning process once again.

The details, shapes, dimensions and other accessory elements, as well as the materials used to manufacture the tools of the invention may be conveniently replaced with others that are technically equivalent and do not depart from the essence of or the scope defined in the claims included below:

## Claims

1. A method for cleaning dissolution vessels (6) and subsequent dosing of a dissolution media **characterised in that** it comprises the following stages:
A. General conditioning, which in turn comprises the successive or simultaneous sub-stages:
a. Overheated water steam generation.
b. Preheating the dissolution media to be dosed
c. Emptying of the waste tank (14)
B. Cleaning the dissolution vessel (6), which in turn comprises the following successive or simultaneous sub-stages:
a. Fitting and adjusting the cover of the dissolution vessel (6)
b. Pressing the button to start the process
c. Aspiration of the dissolution media through the aspiration tube (8).
d. Transferring into the waste tank (14)
e. Injection of water steam at predetermined temperatures and pressure levels for a given time period depending on the dissolution media used and the therapeutic agent tested
f. Aspirating steam using the aspiration tube (8) and transferring into the waste tank (14).
g. Transferring the dissolution media and steam contained in the waste tank to the outside.

2. The method for cleaning dissolution vessels (6) and subsequent dosing of a dissolution media according to claim 1, **characterised in that** after the end of stage B, described in claim 1, the following stage is carried out:
C: Dosing, which in turn comprises the successive or simultaneous sub-stages:
a. Transferring of the preheated dissolution media from the storage deposit to the dosing deposit (21)
b. Measuring the predetermined amount of dissolution media to be dosed using a gravimetric measuring device
c. Transferring the dissolution media into the dissolution vessel (6).

3. The method for cleaning dissolution vessels (6) and subsequent dosing of a dissolution media, according to any of the previous claims, **characterised in that** one or more chemical additives are added to the overheated steam.

4. A mobile modular cleaning and dosing equipment for the implementation of the method, according to any of the previous claims, **characterised in that** it comprises:
A. A cover module (5), which in turn comprises a cover (7) with coupling and seal-tight adjustment means at the top of the dissolution vessel (6); a vacuum breaker system , being said vacuum breaker system an opening arranged in the cover (7), which enables the insertion of a filter; an aspiration tube (8) of variable length coupled in a seal-tight manner to the cover (7) and connected to the aspiration line (12) of the dissolution media; a check valve suitably arranged in the aspiration tube (8); a particle filter suitably arranged in the aspiration tube (8); a supply line for steam connected to a steam distributor (9) that consists of a piece with upper seals; an inner channel for the circulation of steam and a series of holes arranged perpendicularly to the channel with outer openings; a supply line for the dissolution media and an activation switch.
B. An aspiration module for the dissolution media and steam that comprises a aspiration system connected to a waste tank (14)
C. A water steam generation module
D. A dosing module (2) for the dissolution media that contains a storage deposit to store the dissolution media, a gravimetric measurement device (20) and a dosing deposit (21)
E. A control module with an information screen and data input
F. A transportion module that comprises a frame to which the rest of the modules are coupled, and on which transportation means are suitably arranged
G. Corresponding interconnection and connection tubing, fittings and solenoid valves between the equipment and elements of each module and between the different modules.

5. The mobile modular cleaning and dosing equipment, according to claims 4, **characterised in that** the waste tank (14) is equipped with level sensors and a waste pump with a check valve.

6. The mobile modular cleaning and dosing equipment, according to any of the claims 4 to 5, **characterised in that** the steam generation module (3) comprises a boiler manufactured from stainless steel conveniently equipped with one or more electrical heaters, water level sensor, safety thermostat, pressure/temperature control system, solenoid valves, safety valves, waste breaker system, preferably vent valve type, filling pump (17) of the boiler with its corresponding check valve, water supply deposit to the boiler with a level sensor.

7. The mobile modular cleaning and dosing equipment, according to any of the claims 4 to 6, **characterised in that** the storage deposit for the dissolution media is equipped with level sensors and a heating system with temperature control.

8. The mobile modular cleaning and dosing equipment, according to any of the claims 4 to 7, **characterised in that** the control module controls, at least, the activation of the switch for the cover module (5), the steam pressure, the injection time for the steam in the dissolution vessel (6), the time and cycles to aspirate the dissolution media, the temperature and the amount of the dissolution media to be dosed.

9. The mobile modular cleaning and dosing equipment, according to any of the claims 4 to 8, **characterised in that** the aspiration system is an aspiration pump (13) with a sealing gasket.

10. The mobile modular cleaning and dosing equipment, according to any of the claims 4 to 9, **characterised in that** the pressure/temperature control system for the generation module is carried out via a pressure switch.

11. The mobile modular cleaning and dosing equipment, according to any of the claims 4 to 10, **characterised in that** a device for adding chemical additives to the supply line of the steam generated is suitably arranged in the steam generation module (3).

12. The mobile modular cleaning and dosing equipment, according to any of the claims 4 to 11, **characterised in that** the heating system for the dosing module (2) is one or more electrical heaters with a thermostat.

13. The mobile modular cleaning and dosing equipment, according to any of the claims 4 to 12, **characterised in that** the gravimetric measurement device (20) is a load cell.

14. The mobile modular cleaning and dosing equipment, according to any of the claims 4 to 13, **characterised in that** the different modules that comprise the invention are separated from each other or grouped in different combinations of modules, each module or group being provided with the corresponding frame and transport means thereof.

## Patentansprüche

1. Verfahren zum Reinigen von Auflösungsbehältern (6) und nachfolgendes Dosieren eines Auflösungsmediums, **dadurch gekennzeichnet, dass** das Verfahren die folgenden Stufen umfasst:
A. Allgemeines Konditionieren, was wiederum die nachfolgenden oder gleichzeitigen Unterstufen umfasst:
a. Erzeugung von überhitztem Wasserdampf
b. Vorwärmen des zu dosierenden Auflösungsmediums
c. Leeren des Abfalltanks (14)
B. Reinigen des Auflösungsbehälters (6), was wiederum die nachfolgenden oder gleichzeitigen Unterstufen umfasst:
a. Einsetzen und Einstellen der Abdeckung des Auflösungsbehälters (6)
b. Drücken des Tasters zum Starten des Prozesses
c. Ansaugen des Auflösungsmediums durch das Ansaugrohr (8)
c. Übertragen davon in den Abfalltank (14)
e. Einspritzen von Wasserdampf bei vorbestimmten Temperaturen und Druckpegeln für einen gegebenen Zeitraum, je nach verwendetem Auflösungsmedium und getestetem therapeutischen Wirkstoff
f. Ansaugen von Dampf unter Verwendung des Ansaugrohrs (8) und Übertragen davon in den Abfalltank (14)
g. Übertragen des Auflösungsmediums und des Dampfes, die in dem Abfalltank enthalten sind, nach außen.

2. Verfahren zum Reinigen von Auflösungsbehältern (6) und nachfolgendes Dosieren eines Auflösungsmediums nach Anspruch 1, **dadurch gekennzeichnet, dass** nach dem Ende von Stufe B, wie in Anspruch 1 beschrieben, die folgende Stufe ausgeführt wird:
C. Dosieren, was wiederum die nachfolgenden oder gleichzeitigen Unterstufen umfasst:
a. Übertragen des vorgewärmten Auflösungsmediums aus dem Speicherdepot in das Dosierdepot (21)
b. Abmessen der vorbestimmten Menge an zu dosierendem Auflösungsmedium unter Verwendung einer gravimetrischen Messvorrichtung
c. Übertragen des Auflösungsmediums in den Auflösungsbehälter (6).

3. Verfahren zum Reinigen von Auflösungsbehältern (6) und nachfolgendes Dosieren eines Auflösungsmediums nach einem der vorhergehenden Ansprüche, **dadurch gekennzeichnet, dass** einer oder mehrere chemische Zusätze in den überhitzten Dampf gegeben werden.

4. Mobile modulare Reinigungs- und Dosieranlage für die Implementierung des Verfahrens nach einem der vorherigen Ansprüche, **dadurch gekennzeichnet, dass** diese umfasst:
A. Ein Abdeckmodul (5), das wiederum eine Abdeckung (7) mit Kopplungs- und dichten Einstelleinrichtungen an der Oberseite des Auflösungsbehälters (6) umfasst; ein Vakuumunterbrechersystem, wobei das Vakuumunterbrechersystem eine Öffnung ist, die in der Abdeckung (7) angeordnet ist, die das Einführen eines Filters ermöglicht; ein Ansaugrohr (8) verstellbarer Länge, das dichtend mit der Abdeckung (7) gekoppelt ist und mit der Ansaugleitung (12) des Auflösungsmediums verbunden ist; ein Rückschlagventil, das geeignet in dem Ansaugrohr (8) angeordnet ist; einen Teilchenfilter, der geeignet in dem Ansaugrohr (8) angeordnet ist; eine Zufuhrleitung für Dampf, die mit einem Dampfverteiler (9) verbunden ist, der aus einem Stück mit oberen Dichtungen besteht; einen inneren Kanal für die Zirkulation von Dampf und eine Reihe von Löchern, die senkrecht zu dem Kanal mit den äußeren Öffnungen angeordnet sind; eine Zufuhrleitung für das Auflösungsmedium und einen Aktivierungsschalter;
B. Ein Ansaugmodul für das Auflösungsmedium und den Dampf, das ein Ansaugsystem umfasst, das mit einem Abfalltank (14) verbunden ist
C. Ein Wasserdampferzeugungsmodul
D. Ein Dosiermodul (2) für das Auflösungsmedium, das ein Speicherdepot zum Speichern des Auflösungsmediums, eine gravimetrische Messvorrichtung (20) und ein Dosierdepot (21) enthält
E. Ein Steuermodul mit einem Informationsschirm und einer Dateneingabe
F. Ein Transportmodul, das einen Rahmen umfasst, an dem der Rest der Module gekoppelt ist und an dem die Transporteinrichtungen geeignet angeordnet sind
G. Entsprechende Anschluss- und Verbindungsrohrleitungen, Fassungen und Magnetventile zwischen der Anlage und den Elementen jedes Moduls und zwischen den unterschiedlichen Modulen.

5. Mobile modulare Reinigungs- und Dosieranlage nach Anspruch 4, **dadurch gekennzeichnet, dass** der Abfalltank (14) mit Füllstandsensoren und einer Abfallpumpe mit einem Rückschlagventil ausgestattet ist.

6. Mobile modulare Reinigungs- und Dosieranlage nach einem der Ansprüche 4 bis 5, **dadurch gekennzeichnet, dass** das Dampferzeugungsmodul (3) einen Kessel aus rostfreiem Stahl umfasst, der zweckmäßig mit einer oder mehreren elektrischen Heizungen, einem Wasserstandsensor, einem Sicherheitsthermostat, einem Druck-/Temperaturregelsystem, Magnetventilen, Sicherheitsventilen, einem Abfallunterbrechungssystem vorzugsweise des Entlüftungsventiltyps, einer Füllpumpe (17) des Behälters mit ihrem entsprechenden Rückschlagventil, einem Wasserzufuhrdepot zum Behälter mit einem Füllstandsensor ausgestattet ist.

7. Mobile modulare Reinigungs- und Dosieranlage nach einem der Ansprüche 4 bis 6, **dadurch gekennzeichnet, dass** das Speicherdepot für das Auflösungsmedium mit Füllstandsensoren und einem Heizsystem mit Temperatursteuerung ausgestattet ist.

8. Mobile modulare Reinigungs- und Dosieranlage nach einem der Ansprüche 4 bis 7, **dadurch gekennzeichnet, dass** das Steuermodul mindestens die Aktivierung des Schalters für das Abdeckmodul (5), den Dampfdruck, die Einspritzzeit für den Dampf in den Auflösungsbehälter (6), die Zeit und Zyklen zum Ansaugen des Auflösungsmediums, die Temperatur und die Menge an zu dosierendem Auflösungsmedium steuert.

9. Mobile modulare Reinigungs- und Dosieranlage nach einem der Ansprüche 4 bis 8, **dadurch gekennzeichnet, dass** das Ansaugsystem eine Ansaugpumpe (13) mit einem Dichtungssiegel ist.

10. Mobile modulare Reinigungs- und Dosieranlage nach einem der Ansprüche 4 bis 9, **dadurch gekennzeichnet, dass** das Druck-/Temperatursteuersystem für das Erzeugungsmodul über einen Druckschalter ausgeführt wird.

11. Mobile modulare Reinigungs- und Dosieranlage nach einem der Ansprüche 4 bis 10, **dadurch gekennzeichnet, dass** die Vorrichtung zum Zugeben von chemischen Zusätzen zu der Zufuhrleitung des erzeugten Dampfs geeignet in dem Dampferzeugungsmodul (3) angeordnet ist.

12. Mobile modulare Reinigungs- und Dosieranlage nach einem der Ansprüche 4 bis 11, **dadurch gekennzeichnet, dass** das Heizsystem für das Dosiermodul (2) eine oder mehrere elektrische Heizungen mit einem Thermostat ist.

13. Mobile modulare Reinigungs- und Dosieranlage nach einem der Ansprüche 4 bis 12, **dadurch gekennzeichnet, dass** die gravimetrische Messvorrichtung (20) eine Lastzelle ist.

14. Mobile modulare Reinigungs- und Dosieranlage nach einem der Ansprüche 4 bis 13, **dadurch gekennzeichnet, dass** die unterschiedlichen Module, welche die Erfindung umfasst, voneinander getrennt sind oder in unterschiedlichen Kombinationen von Modulen gruppiert sind, wobei jedes Modul oder Gruppe mit der entsprechenden Rahmen- und Transporteinrichtung dafür bereitgestellt ist.

## Revendications

1. Procédé pour le nettoyage de récipients de dissolution (6) et le dosage ultérieur d'un milieu de dissolution **caractérisé en ce qu'**il comprend les étapes suivantes :
A. Le conditionnement général, qui comprend à son tour les sous-étapes successives ou simultanées :
a. La génération de vapeur d'eau surchauffée.
b. Préchauffer le milieu de dissolution à doser
c. Vider le réservoir à matières (14)
B. Nettoyer le récipient de dissolution (6), qui comprend à son tour les sous-étapes successives ou simultanées suivantes :
a. Poser et ajuster le couvercle du récipient de dissolution (6)
b. Appuyer sur le bouton pour démarrer le processus
c. Aspiration du milieu de dissolution à travers le tube d'aspiration (8).
c. Transférer au réservoir à matières (14)
e. Injection de vapeur d'eau à des températures prédéterminées et à des niveaux de pression pour une période de temps donnée en fonction du milieu de dissolution utilisé et de l'agent thérapeutique testé
f. Aspirer la vapeur en utilisant le tube d'aspiration (8) et la transférer dans le réservoir à matières (14).
g. Transférer le milieu de dissolution et la vapeur contenus dans le réservoir à matières à l'extérieur.

2. Procédé pour le nettoyage de récipients de dissolution (6) et le dosage ultérieur d'un milieu de dissolution selon la revendication 1, **caractérisé en ce qu'**après la fin de l'étape B, décrite dans la revendication 1, l'étape suivante est réalisée :
C : Le dosage, qui comprend à son tour les sous-étapes successives ou simultanées :
a. Le transfert du milieu de dissolution préchauffé du dépôt de stockage au dépôt de dosage (21)
b. La mesure de la quantité prédéterminée de milieu de dissolution à doser en utilisant un dispositif de mesure gravimétrique
c. Le transfert du milieu de dissolution dans le récipient de dissolution (6).

3. Procédé pour le nettoyage de récipients de dissolution (6) et le dosage ultérieur d'un milieu de dissolution, selon l'une quelconque des revendications précédentes, **caractérisé en ce qu'**un ou plusieurs additifs chimiques sont ajoutés à la vapeur surchauffée.

4. Équipement de nettoyage et de dosage modulaire mobile pour la mise en oeuvre du procédé, selon l'une quelconque des revendications précédentes, **caractérisé en ce qu'**il comprend :
A. Un module de couvercle (5), qui à son tour comprend un couvercle (7) avec des moyens d'ajustement de couplage et d'imperméabilisation sur le récipient de dissolution (6) ; un système casse-vide, ledit système casse-vide étant une ouverture disposée dans le couvercle (7), qui permet l'insertion d'un filtre ; un tube d'aspiration (8) de longueur variable couplé de façon hermétique au couvercle (7) et raccordé à la ligne d'aspiration (12) du milieu de dissolution ; un clapet anti-retour disposé convenablement dans le tube d'aspiration (8) ; un filtre à particules disposé convenablement dans le tube d'aspiration (8) ; une ligne d'approvisionnement pour la vapeur raccordée à un distributeur de vapeur (9) qui consiste en une pièce avec des joints supérieurs ; un canal interne pour la circulation de vapeur et un ensemble d'orifices disposés perpendiculairement au canal avec des ouvertures externes ; une ligne d'approvisionnement pour le milieu de dissolution et un interrupteur d'activation.
B. Un module d'aspiration pour le milieu de dissolution et la vapeur qui comprend un système d'aspiration lié à un réservoir à matières (14)
C. Un module de génération de vapeur d'eau
D. Un module de dosage (2) pour le milieu de dissolution qui contient un dépôt de stockage pour stocker le milieu de dissolution, un dispositif de mesure gravimétrique (20) et un dépôt de dosage (21)
E. Un module de contrôle avec un écran informatif et une entrée de données
F. Un module de transport qui comprend un châssis auquel le reste des modules sont couplés, et sur lequel les moyens de transport sont disposés convenablement
G. Interconnexion correspondante et tubes de raccordement, poses et électrovannes entre l'équipement et les éléments de chaque module et entre les différents modules.

5. Équipement de nettoyage et de dosage modulaire mobile, selon la revendication 4, **caractérisé en ce que** le réservoir à matières (14) est équipé de capteurs de niveau et d'une pompe à matières et d'un clapet anti-retour.

6. Équipement de nettoyage et de dosage modulaire mobile, selon l'une quelconque des revendications 4 à 5, **caractérisé en ce que** le module de génération de vapeur (3) comprend une chaudière fabriquée à partir d'acier inoxydable convenablement équipé d'un ou plusieurs chauffages électriques, un capteur du niveau de l'eau, un thermostat de sécurité, un système de contrôle de la pression/température, des électrovannes, des soupapes de sécurité, un système casse-matières, de préférence du type soupape de purge, une pompe de remplissage (17) de la chaudière avec sa vanne anti-retour correspondante, un dépôt d'approvisionnement en eau à la chaudière avec un capteur de niveau.

7. Équipement de nettoyage et de dosage modulaire mobile, selon l'une quelconque des revendications 4 à 6, **caractérisé en ce que** le dépôt de stockage pour le milieu de dissolution est équipé de capteurs de niveau et d'un système de chauffage avec le contrôle de la température.

8. Équipement de nettoyage et de dosage modulaire mobile, selon l'une quelconque des revendications 4 à 7, **caractérisé en ce que** le module de contrôle contrôle, au moins, l'activation de l'interrupteur pour le module de couvercle (5), la pression de vapeur, la période d'injection pour la vapeur dans le récipient de dissolution (6), la période et les cycles pour aspirer le milieu de dissolution, la température et la quantité du milieu de dissolution à doser.

9. Équipement de nettoyage et de dosage modulaire mobile, selon l'une quelconque des revendications 4 à 8, **caractérisé en ce que** le système d'aspiration est une pompe d'aspiration (13) avec un joint d'étanchéité.

10. Équipement de nettoyage et de dosage modulaire mobile, selon l'une quelconque des revendications 4 à 9, **caractérisé en ce que** le système de contrôle de la pression/température pour le module de génération est réalisé par un pressostat.

11. Équipement de nettoyage et de dosage modulaire mobile, selon l'une quelconque des revendications 4 à 10, **caractérisé en ce qu'**un dispositif pour ajouter des additifs chimiques à la ligne d'approvisionnement de la vapeur générée est disposé convenablement dans le module de génération de vapeur (3).

12. Équipement de nettoyage et de dosage modulaire mobile, selon l'une quelconque des revendications 4 à 11, **caractérisé en ce que** le système de chauffage pour le module de dosage (2) est un ou plusieurs chauffages électriques avec un thermostat.

13. Équipement de nettoyage et de dosage modulaire mobile, selon l'une quelconque des revendications 4 à 12, **caractérisé en ce que** le dispositif de mesure gravimétrique (20) est une cellule de charge.

14. Équipement de nettoyage et de dosage modulaire mobile, selon l'une quelconque des revendications 4 à 13, **caractérisé en ce que** les différents modules que comprend l'invention sont séparés les uns des autres ou groupés dans différentes combinaisons de modules, chaque module ou groupe étant pourvu du châssis correspondant et des moyens de transport de celui-ci.
